# EUROPÄISCHE PATENTSCHRIFT

(11) **EP 0 488 142 B1**
(45) Veröffentlichungstag und Bekanntmachung des Hinweises auf die Patenterteilung: **25.01.1995**
(21) Anmeldenummer: 91120118.4
(22) Anmeldetag: 26.11.1991
(51) Int. Cl.: A61K 9/127

(54) **Verfahren zur Verkapselung fester oder flüssiger, lipophiler Wirkstoffe zu diesen Wirkstoff enthaltenden Phospholipid-Liposomen sowie Arzneimittel diese Liposomen enthaltend**
Process for encapsulating solid or liquid lipophilic agents in phospholipid-liposomes and medicaments containing those liposomes
Procédé pour encapsuler des principes actifs lipophiles solides ou liquides dans des liposomes phospholipidiques ainsi que des médicaments contenant ces liposomes

(30) Priorität: 29.11.1990 DE 4038075
(43) Veröffentlichungstag der Anmeldung: 03.06.1992
(73) Patentinhaber: B. Braun Melsungen AG, 34209 Melsungen (DE)
(72) Erfinder: Schmitt, Jürgen, W-6313 Homberg/Ohm 3 (DE); Nehne, Jörg, W-3501 Guxhagen (DE); Feller, Wolfgang, W-3508 Melsungen (DE); Berger, Simone, W-3509 Malsfeld/Dagobertshausen (DE)
(74) Vertreter: von Kreisler, Alek, Dipl.-Chem.

(56) Entgegenhaltungen:
- EP-A- 0 211 647
- EP-A- 0 290 296
- WO-A-90/12565
- PATENT ABSTRACTS OF JAPAN vol. 9, no. 119 (C-282)(1842) 23. Mai 1985
- PATENT ABSTRACTS OF JAPAN vol. 13, no. 101 (C-574)(3449) 9. März 1989
- PATENT ABSTRACTS OF JAPAN vol. 12, no. 372 (C-533)(3219) 5. Oktober 1988

## Beschreibung

Die Erfindung betrifft ein Verfahren zur Verkapselung fester oder flüssiger, lipophiler Wirkstoffe zu diesen Wirkstoff enthaltenden Phospholipid-Liposomen aus einem Phospholipid oder Phospholipid-Gemisch mit mindestens einem lipophilen Wirkstoff sowie Arzneimittel, die diese Liposomen enthalten.

Liposomen sind Lipidvesikel, bei denen ein wäßriger Kern von einem oder mehreren Phospholipid-Bilayern umschlossen wird. Seit ihrer Entdeckung durch Bangham (J. Mol. Biol. 13, (1965) 238 - 252) wurden sie sehr eingehend untersucht. Dabei war zunächst vor allen Dingen ihre strukturelle Ähnlichkeit mit den biologischen Membranen von besonderem Interesse.
Erst im Laufe der letzten 15 Jahre rückten die pharmazeutisch und medizinisch nutzbaren Eigenschaften der Liposomen mehr in den Vordergrund.
Man teilt heute allgemein die Liposomen aufgrund ihrer Struktur und ihrer Teilchengröße in drei verschiedene Gruppen ein:

| | |
|---|---|
| 1. Multilamellar vesicles (MLV) | 0,5 - mehrere »m |
| 2. small unillamelar vesicles (SUV) | 0,02 - 0,05 »m |
| 3. large unilamellar vesicles (LUV) | 0,06 - 0,5 »m |

Für jede einzelne Liposomenart gibt es unterschiedliche Herstellungsmethoden, eine Übersicht über Vor- und Nachteile der einzelnen Methode gibt Szoka (Anm. Rev. Biophys. Bioeng., (1980) 9, 467) und Gregoriadis (Liposomes as Drug Carriers, Wiley and Sons, New York 1984).

Nicht alle bekannten Herstellungsmethoden sind geeignet, Liposomen in pharmazeutisch akzeptabler Qualität und entsprechender Menge herzustellen. Zu den wichtigsten Methoden zählen Homogenisierungstechniken, z.T. unter dem Einsatz spezieller Maschinen wie Hochdruckhomogenisatoren, und verschiedene Extrusionstechniken, durch den Einsatz unterschiedlich gearteter Filter.

Der strukturelle Aufbau der Liposomen ermöglicht es, sie als Transportvehikel für Wirkstoffe zu benutzen (Knight, C. G. ed., Research monographs in cell and tissue physiology, Vol. 7, Liposomes: From physical structure to therapeutic applications, Elsevier/North-Holland, Biomedical Press, Amsterdam, New York, Oxford 1981). Aufgrund der Vesikelstruktur und der amphiphilen Natur der Phospholipide können wasserlösliche Wirkstoffe in den wässrigen Kern und lipophile Wirkstoffe in das Bilayer der Liposomen eingeschlossen werden.

Das Anwendungsspektrum der Liposomen als Wirkstoffträger ist sehr breit. Bei parenteraler Anwendung (i.v., s.c., i.m., i.p.) kann ein Schutz des Wirkstoffs vor Abbau, das Anlegen eines Wirkstoffdepots und eine gezielte Wirkstofffreisetzung erreicht werden [Mihalko, P. I., Schreier, H., Abra, R. M., Liposomes as Drug Carriers (G. Gregoriadis, ed.), Wiley and Sons, New York 1988]. Zusätzlich kann durch den Einsatz wirkstoffbeladener Liposomen die Gewebeverteilung des Wirkstoffes verändert werden, wodurch sich zusätzliche therapeutische Vorteile ergeben.

Wirkstoffbeladene Liposomen eignen sich ebenfalls zur Wirkstoffaufnahme durch den Respirationstrakt und zur topischen Applikation (Gregoriadis, G. ed., Liposomes as Drug Carriers, Wiley and Sons, New York 1988; Uster, P.S., Specialized Vehicles for Topical Delivery, (D. T. Amman, eds.), Marcel Dekker, New York 1989). In unterschiedlichsten Therapiebereichen sind wirkstoffhaltige Liposomen in Tierexperimenten und klinischen Untersuchungen eingesetzt worden (Schenk et al., Internationale Trends in der Entwicklung von Liposomen unter besonderer Berücksichtigung der Patentsituation, Pharmazie 43, (1988) 457 - 463); Fichtner u. Arndt, Stand und Perspektiven der Liposomenforschung, Pharmazie 44, (1989) 752 - 757)]. Am intensivsten wurde der Einsatz von Liposomen in antineoplastischen, antimikrobiellen und antiviralen Therapien untersucht.

Entsprechend diesen Schwerpunkten ist die Verkapselung von Wirkstoffen wie z. B. Doxcrubincin (US-PS-4 460 577; Sculier et al., Eur. J. Cancer 24, 527, 1988), Adriamycin (DE-OS-28 42 608), Amphotericin B (Lopez-Berestein 1986), Interleukin 2 (GB-2 157 172, Anderson 1988), und Na-Stibogluconat (EP-A-0 072 234, Carter et al. 1988) mit unterschiedlichen Herstellungtechniken besonders gut dokumentiert. Zu den oben erwähnten Wirkstoffen und Wirkstoffgruppen zählen sowohl wasserlösliche als auch lipidlösliche Substanzen, so daß bei der Herstellung sehr unterschiedliche Gesichtspunkte berücksichtigt werden müssen.

Im Einzelnen beschreibt US-PS-4 460 577 ein Verfahren, um nicht in Liposomen eingeschlossene Wirkstoffe aus einer Liposomen-Suspension zu entfernen. Hierzu wird diese Suspension mit einem Ionenaustauscherharz in Kontakt gebracht, welches den Wirkstoff selektiv binded. Das Harz muß anschließend wieder von der Suspension getrennt werden.

DE-OS-28 42 608 beschreibt ebenfalls ein Verfahren zur Reinigung von Liposomen-Suspensionen von nicht eingeschlossenen Heilmitteln (Wirkstoff) mit Hilfe von Ionenaustauscherharzen.

GB-2 157 172 beschreibt ein Verfahren zur Herstellung von Interleukin enthaltenden Liposomen durch Auflösung eines Interleukins und eines Lipids (z.B. Lecithin und Cholesterol) in einem geeigneten organischen Lösungsmittel (z.B. Chloroform). Das Lösungsmittel wird anschließend entfernt und der Rückstand in einer Pufferlösung suspendiert. Die eigentlichen wirkstoffhaltigen Liposomen werden danach durch Rühren und Homogenisieren der Suspension und anschließendem Isolieren der Liposomen erhalten.

EP-A-0 072 234 beschreibt ein Verfahren zur Herstellung einer Liposomen-Wirkstoff-Formulierung, bei welchem die Lipidkomponente zunächst in einem organischen Lösungsmittel gelöst wird und die so erhaltene Lösung in einer die Wirkstoffkomponente enthaltenden wässrigen Lösung emulgiert wird. Das organische Lösungsmittel wird anschließend verdampft.

EP-A-0 290 296 und WO-A-9 012 565 betreffen Verfahren zur Herstellung von Liposomen.

Bei der Verkapselung wasserlöslicher Wirkstoffe spielt das Verkapselungsvolumen, primär abhängig vom mittleren Teilchendurchmesser der Liposomen, und die Freisetzungsrate des Wirkstoffs, abhängig von der Struktur des Phospholipid-Bilayers, eine wesentliche Rolle. Die Größe der Liposomen wird auch durch deren Einsatzgebiet mit bestimmt, bei parenteraler Verwendung müssen die Liposomen-Dispersionen sterilisierbar sein. Die einzige für Liposomen geeignete Sterilisationsmethode ist die Filtration durch 0,22 »m-Sterilfilter, daraus ergibt sich, daß die Liposomen einen mittleren Teilchendurchmesser von 200 nm nicht überschreiten dürfen.

Beim Einschluß lipidlöslicher Substanzen muß der Wirkstoff so im Phospholipid-Bilayer verteilt werden, daß die strukturelle Stabilität des Bilayers erhalten bleibt. Die Vermischung des Phospholipids mit dem lipophilen Wirkstoff wird dabei unter dem Einsatz von organischen Lösungsmitteln und/oder Detergentien vorgenommen.

Bei der liposomalen Verkapselung lipophiler, wasserunlöslicher Wirkstoffe werden nach dem heutigen Kenntnisstand demnach grundsätzlich organische Lösungsmittel, wie z.B. Chloroform oder Ethanol, oder unterschiedliche Detergentien (z.B. Gallensalze, Octylglukosid) eingesetzt, um die Phospholipide mit dem jeweiligen Wirkstoff zu mischen. Das hat für die Herstellung und Verwendung der Liposomen mehrere Nachteile. Zum einen wird der gesamte Herstellungsprozeß recht langwierig und technisch aufwendig, denn es müssen zunächst die Phospholipide und der Wirkstoff in dem entsprechenden organischen Lösungsmittel gelöst werden und dann miteinander in einem bestimmten Verhältnis gemischt werden. Die Entfernung des Lösungsmittels wird meistens im Vakuum vorgenommen, worauf sich noch ein mehrstündiges Trocknen des Lipidfilms im Hochvakuum, zur Entfernung des Restlösungsmittels, anschließt. Danach kann erst mit dem eigentlichen Herstellungsprozeß begonnen werden. Zum anderen muß damit gerechnet werden, daß Lösungsmittelreste in der fertigen Liposomen-Dispersion zurückbleiben, was eine zusätzliche Analytik verlangt und im Falle von chlorierten Kohlenwasserstoffen und anderen toxischen Lösungsmitteln zu Komplikationen führen kann. Neben diesen beiden wichtigen Aspekten ist es bei dieser Art der Herstellung nur unter erheblichem apparativem Aufwand und hohen Kosten möglich, wirkstoffhaltige Liposomen im Maßstab von mehreren Litern herzustellen. Dies gilt insbesondere beim Einsatz von Detergentien, wobei eine besondere Herstellungstechnik (Detergensdialyse) zum Einsatz kommt, wodurch neben dem "Rest-Detergens-Gehalt", zusätzlich noch die eingeschränkte Einsetzbarkeit von Phospholipiden und Wirkstoffen hinzukommt.

Das der Erfindung zugrundeliegende Problem besteht demnach in der Zurverfügungstellung eines einfachen Verfahrens zur Einkapselung fester oder flüssiger, lipophiler Wirkstoffe in Phospholipid-Liposomen unter Verwendung physiologisch unbedenklicher Rohstoffe. Die lipophilen Wirkstoffe sollten ohne Einsatz eines organischen Lösungsmittels und/oder Detergentien nur durch Verwendung geeigneter Phospholipide und gegebenenfalls lipidhaltiger Hilfsstoffe in eine wasserlösliche und parenteral, insbesondere intravenös applizierbare Form überführt werden.

Das Problem wurde erfindungsgemäß gelöst durch ein Verfahren zur Verkapselung fester oder flüssiger lipophiler Wirkstoffe zu diesen Wirkstoff enthaltenden Phospholipid-Liposomen aus einem Phospholipid oder Phospholipid-Gemisch mit mindestens einem lipophilen Wirkstoff in einem Hydratisierungsmedium, umfassend einen ersten Schritt, in dem man multilamellare, wirkstoffhaltige Liposomen erzeugt und einen zweiten Schritt, in dem man die mittlere Größe der Wirkstoff enthaltenden Phospholipid-Liposomen durch übliche Verfahren auf eine bestimmte Größe einstellt, dadurch gekennzeichnet, daß man in dem ersten Schritt das Phospholipid oder Phospholipid-Gemisch unter Rühren in der Hälfte bis einem Dreiviertel der Menge des Hydratisierungsmediums unter Schutzgas dispergiert und nach Beendigung dieses Vorgangs auf eine Temperatur oberhalb der Phasenübergangstemperatur des Phospholipids oder Phospholipid-Gemisches temperiert, bei welcher man den Wirkstoff der Dispersion zugibt sowie den noch verbliebenen Teil des Hydratisierungsmediums auffüllt und man anschließend noch so lange bei dieser Temperatur weiterrührt, bis durch übliche Analysenmethoden kein freier Wirkstoff im Hydratisierungsmedium mehr nachgewiesen werden kann, wobei das Hydratisierungsmedium frei von organischen Lösungsmitteln und/oder Detergentien ist.

Das Phospholipid oder Phospholipid-Gemisch und/oder der Wirkstoff kann in Gegenwart eines Hilfsstoffes dispergiert werden.

Der Vorteil des erfindungsgemäßen Verfahrens besteht darin, daß lipophile, wasserunlösliche Wirkstoffe ohne die Verwendung organischer Lösungsmittel oder Detergentien in das Bilayer von Phospholipid-Liposomen eingearbeitet werden können. Die lipophilen Wirkstoffe können somit unter dem Einsatz physiologisch unbedenklicher Rohstoffe in eine wasserlösliche und parenteral, insbesondere intravenös applizierbare Form überführt werden.

Man kann das gesamte Herstellungsverfahren in zwei Schritte aufteilen: Dispergierung von Wirkstoff und Lipid und damit die Herstellung multilamellarer, wirkstoffhaltiger Liposomen in einem ersten Schritt und in einem zweiten Schritt "Sizing" der im ersten Schritt hergestellten multilamellaren Liposomen in den jeweils gewünschten Größenbereich und damit Herstellung unilamellarer Liposomen.

Im zweiten Schritt können im Prinzip alle bekannten Methoden angewandt werden, insbesondere die Hochdruckhomogenisierung, die Extrusion durch Polycarbonatmembranen, die Mikrofluidisierung oder eine Methodenkombination. Sinnvolle Kombinationen von verschiedenen Methoden stellen z.B. die Hochdruckhomogenisierung mit anschließender Extrusion durch Keramikfilter oder PC-Membranen oder die Mikrofluidisierung mit anschließender Extrusion durch Keramikfilter oder PC-Membranen dar.

Werden die oben angeführten Herstellungsmethoden benutzt, entstehen hauptsächlich unilamellare Liposomen, deren Teilchengröße durch Variation der unterschiedlichen Herstellungsparameter (z.B. Druck, Temperatur, Anzahl der Bearbeitungsdurchgänge) eingestellt werden kann.

Grundsätzlich können somit, in Abhängigkeit vom zweiten Schritt, Liposomen in allen den Größen hergestellt werden, die für die parenterale Applikation geeignet sind. Für die intravenöse Applikation ist jedoch eine Größe von < 200 nm bevorzugt.

Im folgenden wird die Dispergierung von Wirkstoff und Lipid und die Bildung der multilamellaren Liposomen allgemein beschrieben, wobei besonders die Reihenfolge der Einzelschritte von entscheidender Bedeutung ist.

Eine entsprechende Menge Phospholipid oder Phospholipid-Gemisch wird abgewogen und gegebenenfalls zusammen mit Hilfsstoffen in einen Laborreaktor überführt. Der Laborreaktor ist bestückt mit einem Rührwerkzeug, einem Ultraturrax und einer Temperiervorrichtung. Das Reaktorgefäß wird mit Schutzgas (z.B. Argon oder Stickstoff) begast und danach werden bevorzugt die Hälfte bis ein Dreiviertel des für die eingewogene Lipidmenge berechneten Volumens an Hydratisierungsmedium zugegeben. Rührwerkzeug und Ultraturrax werden in Betrieb gesetzt und das Phospholipid in dem Hydratisierungsmedium dispergiert und dabei kontinuierlich mit Schutzgas begast. Dieser Vorgang ist nach maximal 30 Minuten beendet. Der Ultraturrax wird nun ausgeschaltet und die Dispersion wird auf eine Temperatur, die oberhalb der Phasenübergangstemperatur des jeweiligen Phospholipids oder Phospholipid-Gemisches liegt, vorzugsweise auf eine Temperatur zwischen 10 und 70°C, temperiert. Ist die gewünschte Temperatur erreicht, wird der entsprechende Wirkstoff oder das Wirkstoffgemisch gegebenenfalls zusammen mit einer definierten Menge eines Hilfsstoffes, je nach Konsistenz portionsweise oder mit Hilfe einer peristaltischen Pumpe in die Lipiddispersion eingebracht. Hierbei wird mit einer Geschwindigkeit von mindestens 50 U/min gerührt und die Dispersion weiterhin mit Schutzgas überschichtet. Die Wirkstoffzugabe sollte langsam erfolgen. Nach Beendigung der Zugabe wird die Dispersion mit Hydratisierungsmedium auf das gewünschte Endvolumen aufgefüllt und unter Beibehaltung von Rührergeschwindigkeit und Temperatur weiterbehandelt. In größeren zeitlichen Abständen wird aus der Dispersion eine Probe entnommen (ungefähr 5 ml), und die Verteilung des Wirkstoffs im Phospholipid untersucht.

Verschiedene Untersuchungsmethoden stehen dafür zur Auswahl und können je nach physikalischer Beschaffenheit des lipophilen Wirkstoffes (fest, flüssig) angewandt werden. Es sind dies insbesondere die Zentrifugation (30 min bei 4000 xg), die lichtmikroskopische Untersuchung (x 312) oder die Extrusion durch eine Polycarbonat-Membran (Porendurchmesser 0,1 »m oder kleiner).

Durch die Zentrifugation kann festgestellt werden, ob sich freier Wirkstoff sedimentieren läßt, bzw. aufrahmt, also noch nicht homogen im Phospholipid dispergiert ist. Werden bei der lichtmikroskopischen Untersuchtung unstrukturierte Tröpfchen oder Partikel bzw. kristalline Partikel beobachtet, ist das ebenfalls ein Indiz dafür, daß der Wirkstoff noch nicht vollständig in den Phospholipid-Bilayern verteilt ist. Bei der Extrusion durch Polycarbonat-Membranen (nur feste Wirkstoffe) wird der noch nicht im Bilayer eingeschlossene Wirkstoff auf der Membranoberfläche zurückgehalten.

Die Dispergierung wird erst dann beendet, wenn mit den oben beschriebenen Methoden kein "freier" Wirkstoff mehr nachzuweisen ist. Temperatur und Dauer der Dispergierung müssen im Einzelfall auf den Wikstoff und die eingesetzten Lipide und sonstigen Hilfsstoffe abgestimmt werden. Die Temperatur sollte aber in jedem Fall, wie bereits erwähnt, oberhalb der Phasenübergangstemperatur T_{c} des eingesetzten Phospholipids liegen. Nach Beendigung der Dispergierung können mit einer der oben beschriebenen herkömmlichen Methoden die multilamellaren, wirkstoffhaltigen Liposomen in unilamellare Liposomen gewünschter Größe überführt werden.

Die eingesetzten Mengenverhältnisse von Phospholipid, Hydratisierungsmedium und Wirkstoff sind von der gestellten Aufgabe abhängig. Grenzen sind jedoch durch die physiologische Verträglichkeit sowie durch die Viskosität der am Ende erhaltenen Wirkstoff enthaltenden Liposomen-Dispersion gegeben.

Anhand der unten aufgeführten Beispiele ist zu erkennen, daß mit dieser Methode eine Vielzahl unterschiedlicher, lipophiler Wirkstoffe liposomal verkapselt werden können. Die experimentellen Details bei der Dispergierung wie Temperatur, Dispergierzeit, Dauer der Wirkstoffzugabe, müssen dabei für den jeweiligen Wirkstoff in Kombination mit dem ausgewählten Phospholipid ausgetestet werden. Bei Anwendung mehrerer Wirkstoffe und/oder mehrerer Phospholipide sind entsprechend mehr Parameter zu berücksichtigen. Das gleiche gilt für den Einsatz von zusätzlichen Hilfsstoffen für die Phospholipide und/oder Wirkstoffe. Bei dem der Erfindung zugrundeliegenden Verfahren können alle üblicherweise zur Liposomen-Herstellung eingesetzten Phospholipide oder Phospholipid-Gemische benutzt werden. Etwaige Einschränkungen hinsichtlich der Phospholipidauswahl ergeben sich höchstens durch die Art des zu verkapselnden Wirkstoffs oder Wirkstoffgemisches. Im einzelnen können natürliche, halbsynthetische oder synthetische Phospholipide und/oder Phospholipid-Gemische zur Verkapselung lipophiler Wirkstoffe mit der beschriebenen Methode eingesetzt werden.

Eingesetzt werden können natürliche Phospholipide und Phospholipid-Gemische aus Soja oder Ei, so z.B. Gemische unterschiedlicher Zusammensetzung aus Phosphatidylcholin und Phosphatidylethanolamin mit einem geringen Anteil anderer Lipide oder Lipidklassen, wie z.B. neutrale Lipide, Glykolipide und Sphingomyeline.

Weitere Phospholipide natürlichen Ursprungs aus anderen biologischen Materialien sind Phosphatidylcholin (PC), Phosphatidylethanolamin (PE), Phosphatidylglycerol (PG), Phosphatidylinosit (PJ), Phosphatidylserin (PS), Phosphatidsäure (PA).

Die vorstehend aufgeführten Lipide können des weitern durch katalytische Hydrierung chemisch modifiziert werden und in unterschiedlichen Hydrierungsgraden Verwendung finden.

Synthetische und halbsynthetische Phospholipide mit definierter Fettsäurezusammensetzung, wie z.B. Di-palmitoyl-phosphatidylcholin (DPPC), Di-stearoyl-phosphatidylcholin (DSPC), Di-palmitoyl-phosphatidylethanolamin (DPPE), Di-stearoylphosphatidylethanolamin (DSPE), Di-myristoyl-phosphatidylserin (DMPS), Di-oleyl-phosphatidylcholin (DOPC) und alle physikalisch möglichen Mischungen der einzelnen Substanzen.

Prinzipiell können alle lipophilen Wirkstoffe oder deren Gemische mit Hilfe der beschriebenen Methode liposomal verkapselt werden. Voraussetzung ist die exakte Ausarbeitung der für das jeweilige Wirkstoff/Lipid-Gemisch optimalen Versuchsbedingungen, auf die oben beschriebene Art und Weise. Substanzen aus den folgenden Wirkstoffgruppen bieten sich für eine liposomale Verkapselung nach der der Erfindung zugrunde liegenden Methode insbesondere an:
a) Antibiotika (z.B. Gentamycin)
b) Hormone / Steroide (z.B. Testosteron)
c) Fungizide (z.B. Amphotericin B)
d) Tranquilizer (z.B. Diazepam)
e) Antineoplastische Substanzen / Immunsuppressiva (z.B. Cyclosporin A)
f) Anaesthetika / Analgetika (z.B. Propanidid, Fentanyl).

Zusätzlich zu den oben aufgeführten Phospholipiden und Wirkstoffen können bei dem erfindungsgemäßen Verfahren andere Lipide und lipidartige Substanzen als Hilfsstoffe eingesetzt werden. Dazu gehören C₄-C₂₀-Fettsäure-C₁-C₄-alkylester oder C₄-C₂₂-fettsäure-C₁-C₄-alkylester, die sich in Bilayer einbauen lassen, z.B. Ölsäure- und Linolsäureethylester, und/oder die als Antioxidantien wirkenden Tocopherole, Tocopherolester und Ascorbylpalmitat, und/oder Stearylamin und Dicetylphosphat, die zur Erzeugung einer Oberflächenladung auf den Liposomen eingesetzt werden können und/oder Verbindungen zur Stabilisierung des Bilayers, wie z.B. Cholesterin oder dessen Derivate.

Als wäßrige Phase bzw. Hydratisierungsmedien können alle zur Erzeugung einer physiologisch verträglichen, isotonischen Lösung üblicherweise eingesetzten Substanzen benutzt werden, z.B. physiologische Kochsalzlösung, 2,5 %ige Glycerinlösung, 5 %ige Xylitlösung. Um einen pH-Wert im physiologisch verträglichen Bereich zu erzeugen, können auch Puffer zugesetzt werden, z.B. Tris-(hydroxymethyl)-aminomethan.

Die nach dem erfindungsgemäßen Verfahren hergestellten wirkstoffhaltigen Liposomen-Dispersionen in Arzneimitteln können insbesondere in der antineoplastischen, antimikrobiellen und antiviralen Therapie verwendet werden. Dementsprechend betrifft die vorliegende Erfindung insbesondere Arzneimittel, die wirkstoffenthaltende Phospholipid-Liposomen enthalten, die nach dem erfindungsgemäßen Verfahren hergestellt wurden. Als Wirkstoffe kommen insbesondere die oben angeführten Wirkstoffe in Betracht.

Das erfindungsgemäße Verfahren zur Herstellung wirkstoffhaltiger Liposomen-Dispersionen kann insbesondere zur Herstellung Propanidid-haltiger Liposomen-Dispersionen eingesetzt werden. Diese Dispersionen finden Anwendung als Mittel zur Narkoseeinleitung und/oder Kurzzeit-Narkose.

Durch die erfindungsgemäße liposomale Verkapselung lipophiler Wirkstoffe werden deren Eigenschaften und Wirkungen nicht beeinträchtigt oder verändert. Auch die durch liposomalen Einschluß erreichbaren, bekannten Vorteile, wie z.B. bessere Wirksamkeit, gezielte Wirkstoffabgabe, höhere lokale Wirkstoffkonzentration, bleiben bei Anwendung des erfindungsgemäßen Verfahrens erhalten.

Der wesentliche Vorteil des in der Erfindung beschriebenen Verfahrens zur Herstellung wirkstoffhaltiger Liposomen durch Verkapselung lipophiler, d.h. schwer oder praktisch nicht wasserlöslicher Wirkstoffe, liegt darin, daß bei der Liposomenherstellung keine organischen Lösungsmittel oder Detergentien eingesetzt werden. Dadurch wird zum einen der Herstellungsprozeß vereinfacht, zum anderen wird die Produktsicherheit und die Verträglichkeit der Wirkstoffzubereitungen verbessert, weil toxische Effekte und anaphylaktoide Reaktionen, hervorgerufen durch Restlösungsmittel und Detergentien, vermieden werden können.

Die nachfolgenden Beispiele sollen das erfindungsgemäße Verfahren sowie die Verwendung der danach erhaltenen wirkstoffhaltigen Liposomen-Dispersionen verdeutlichen, ohne darauf beschränkt zu sein.

### 1. Beispiel: Herstellung von Propanidid-Liposomen

### a) Dispergierung und Herstellung multilamellarer Propanidid-Liposomen

60 g Eiphospholipide werden abgewogen und in das Reaktorgefäß eines Laborreaktors (Modell LR-A 1000, Fa. Janke und Kunkel), ausgerüstet mit einem Flügelrührer und einem Ultraturrax, überführt. Das Reaktorgefäß wird mit Hilfe eines Wasserbades (z.B. Thermomix ^{R}/Frigomix ^{R} -Kombination, Fa. B. Braun Melsungen AG) auf 20 - 25°C thermostatisiert. Dazu werden 200 ml Hydratisierungsmedium (10 mM Tris-(hydroxymethyl)-aminomethan . HCl in 2,5 % Glycerin-Lösung, pH 7,5) gegeben. Rührer und Dispergierwerkzeug werden in die Flüssigkeit abgesenkt, der Laborreaktor wird verschlossen und ein Stickstoffstrom wird über die Flüssigkeit geleitet. Rührer und Ultraturrax werden in Betrieb genommen (Geschwindigkeiten: Rührer 20 U/min, Ultraturrax 8000 U/min) und das Phospholipid über einen Zeitraum von 15 min dispergiert. Danach wird das Reaktorgefäß auf 50°C temperiert.

8 g Propanidid und 6 g Linolsäureethylester werden in ein 50 ml Becherglas eingewogen und miteinander vermischt. Diese Mischung wird mittels einer Peristaltikpumpe (z.B. Infusomat ^{R}, Fa. B. Braun Melsungen AG) in die Phospholipid-Dispersion gepumpt.

Dieser Vorgang dauert genau 1 Stunde, während dieser Zeit wird die Dispersion nur gerührt (20 U/min). Nachdem die Zugabe des Propanidid/Linolsäureethylester-Gemisches beendet ist, wird die Dispersion 24 Std. unter Beibehaltung der Rührergeschwindigkeit (20 U/min) und der Temperatur (50°C) gerührt. Anschließend wird mit Hydratisierungsmedium auf ein Volumen von 400 ml aufgefüllt.

### b) Herstellung überwiegend unilamellarer Propanidid-Liposomen

Die nach a) hergestellte Propanidid-haltige Phospholipid-Dispersion wird in einen Mikrofluidizer (Modell 110, Fa. Microfluidics) gegeben und 2 Std. bei 750 bar homogenisiert. Produktausgang und Vorlagegefäß des Mikrofluidizers werden mit einem Thermostaten auf 20°C temperiert. Die fertige Liposomen-Dispersion wird durch 0,2 »m-Sterilfilter (Fa. Millipore) filtriert. Mittlerer Teilchendurchmesser und Polydispersität der Dispersion werden nach der Laserstreulicht-Meßmethode, z.B. mit dem Autosizer II, der Fa. Malvern, bestimmt.

### Ergebnis:

Mittlerer Teilchendurchmesser: 65 nm
Polydispersität: > 0,1.

Der Propanididgehalt der Liposomen wird mittels HPLC bestimmt und beträgt 19,2 mg/ml Dispersion. Die Dispersion ist opaleszent und leicht bräunlich gefärbt.

### 2. Beispiel: Herstellung von Propanidid-Liposomen

### a) Dispergierung

Wird analog zur Beschreibung in 1a) durchgeführt.

### b) Herstellung

Die Propanidid-Phospholipid-Dispersion wird in das Vorratsgefäß eines Hochdruckhomogenisators mit LW-Ventilanordnung (z.B. Fa. Rannie) überführt und 20 Minuten bei einem Druck von 600 bar homogenisiert. Produktauslaß und Vorlagegefäß werden auch hier auf 20°C temperiert. Von der sterilfiltrierten Liposomen-Dispersion werden mittlerer Teilchendurchmesser und der Propanidid-Gehalt bestimmt:

### Ergebnis:

Mittlerer Teilchendurchmesser: 85 nm
Polydispersität: 0,2
Propanididgehalt: 18,5 mg/ml Liposomen-Dispersion

### 3. Beispiel: Herstellung von Propanidid-Liposomen

### a) Dispergierung:

Durchführung wie in 1a).

### b) Herstellung:

Die Propanidid-Phospholipid-Dispersion wird in einen handelsüblichen Extruder (Fa. Lipex Biomembranes, Inc.) eingefüllt und bei 25 bar jeweils fünfmal durch Polycarbonat-Membranen (Fa. Nuclepore) mit den Porenweiten 400, 200 und 100 nm extrudiert. Der Extruder wird mit Hilfe eines Wasserbades auf 20°C temperiert. Nach Sterilfiltration werden folgende Analysenergebnisse erhalten:
Mittlerer Teilchendurchmesser: 115 nm
Polydispersität: > 0,1
Propanididgehalt: 19,0 mg/ml Liposomen-Dispersion

### 4. Beispiel: Herstellung von Propanidid-Liposomen

### a) Dispergierung:

Durchführung wie in 1a).

### b) Herstellung:

Mikrofluidisierung wie in 1b) beschrieben, mit dem Unterschied, daß nur 15 min. lang in der angegebenen Art und Weise homogenisiert wird, danach wird die Dispersion entweder
1. 10 mal durch einen Keramikfilter (Ceraflo ^{R}, Fa. Millipore) der Porenweite 0,2 »m mit einem Druck von 14 bar gepreßt. Die Liposomen-Dispersion wird dabei mit Hilfe eines Thermostaten auf 20°C temperiert.
**oder**
2. 12 mal durch einen Extruder (Fa. Lipex Biomembranes Inc.) bestückt mit einer Polycarbonat-Membran (Fa. Nuclepore) der Porenweite 80 nm mit einem Druck von 25 bar, bei 20°C filtriert.

Nach Sterilfiltration werden für die beiden Produkte die folgenden Analysendaten erhalten:

| | | |
|---|---|---|
| 1. | Mittlerer Teilchendurchmesser | 143 nm |
| | Polydispersität | 0,15 |
| | Propanididgehalt | 18,7 mg/ml Dispersion |
| 2. | Mittlerer Teilchendurchmesser | 93 nm |
| | Polydispersität | 0,08 |
| | Propanididgehalt | 19,2 mg/ml Dispersion |

### 5. Beispiel: Herstellung von Propanidid-Liposomen

### a) Dispergierung:

Durchführung wie in 1a)

### b) Herstellung:

Hochdruckhomogenisierung wie unter 2b) beschrieben, mit dem Unterschied, daß nur 5 min. lang homogenisiert wird. Danach wird die Dispersion genau wie im 4. Beispiel unter 1. und 2. beschrieben weiterbehandelt.

Nach Sterilfiltration werden die folgenden analytischen Daten ermittelt:

| | | |
|---|---|---|
| 1. | Mittlerer Teilchendurchmesser | 151 nm |
| | Polydispersität | 0,21 |
| | Propanididgehalt | 19,0 mg/ml Dispersion |
| 2. | Mittlerer Teilchendurchmesser | 88 nm |
| | Polydispersität | 0,07 |
| | Propanididgehalt | 18,4 mg/ml Dispersion |

### 6. Beispiel: Verwendung der in den Beispielen 1 - 5 hergestellten Propanidid-Liposomen

Propanidid-Liposomen, hergestellt gemäß den Beispielen 1 - 5, wurden 3 Hunden intravenös appliziert. Wie aus der Literatur über andere galenische Zubereitungen von Propanidid bekannt, wurde eine Dosierung von 40 mg Propanidid/kg KG, hier jedoch in liposomaler Form, gewählt:

| **Tier-Nr.** | **kg KG** | **Injekt.-Vol. (ml)** | **Injekt.-Dauer (min.)** |
|---|---|---|---|
| 1 | 24,8 | 49,6 | 6,2 |
| 2 | 23,4 | 46,8 | 5,8 |
| 3 | 18,5 | 37,0 | 4,6 |

Bei allen Tieren trat eine Narkose ein, bei der der Lid- und Cornealreflex erhalten blieben:

| **Tier-Nr.** | **Beginn der Wirkung (sec.)** | **Rückkehr Zungenreflex (min.)** | **Beginn Orientierung (min.)** |
|---|---|---|---|
| 1 | 90 | 11,0 | 13,25 |
| 2 | 45 | 7,3 | 8,3 |
| 3 | 60 | 7,0 | 12,0 |

### 7. Beispiel: Herstellung von Fentanyl-haltigen Liposomen

### a) Dispergierung und Herstellung multilamellarer Fentanyl-Liposomen

17 g Eiphospholipide und 3 g Di-palmitoyl-phosphatidylcholin (DPPC) werden in das Reaktorgefäß (temperiert auf 25°C) eines Laborreaktors (Bsp. 1a) eingewogen. Dazu werden 200 ml Hydratisierungsmedium (20 mM Tris-(hydroxymethyl)-aminomethan . HCl in 5 % Sorbit-Lösung, pH 8,0) gegeben. Gemäß Bsp. 1 wird bei folgenden Rührgeschwindigkeiten 15 min. dispergiert: Rührer 20 U/min., Ultraturrax 8000 U/min.

Danach werden 20 mg Fentanylbase in 4 Portionen zu je 5 mg in Abständen von jeweils 20 min. in die Dispersion eingetragen. Während der Wirkstoffzugabe wird die Dispersion mit einer Geschwindigkeit von 20 U/min. gerührt und der Laborreaktor auf 35°C thermostatiert. Nach Beendigung der Wirkstoffzugabe wird die Dispersion noch 4 Stunden unter den erwähnten Bedingungen gehalten. Anschließend wird mit Hydratisierungsmedium auf ein Endvolumen von 400 ml aufgefüllt.

### b) Herstellung überwiegend unilamellarer Fentanyl-Liposomen

Die nach a) hergestellten Fentanyl-haltigen, multilamellaren Liposomen werden mittels Hochdruckhomogenisierung (gemäß Beispiel 2b) in überwiegend unilamellare Liposomen überführt. Eine Analyse der Liposomen-Dispersion nach den üblichen Kriterien ergab folgende Ergebnisse:

| | |
|---|---|
| Mittlerer Teilchendurchmesser | 78 nm |
| Polydispersität | 0,22 |
| Fentanyl-Konzentration (HPLC) | 48,2 »g/ml Dispersion |

### 8. Beispiel: Herstellung von Diazepam-Liposomen

### a) Dispergierung und Herstellung multilamellarer Diazepam-Liposomen

40 g Soja-Phospholipide und 5 g Cholesterin werden in das Reaktorgefäß (temperiert auf 50°C) eines Laborreaktors (Bsp. 1a) eingewogen. Dazu werden 200 ml Hydratisierungsmedium (0,9 % NaCl) gegeben. Es wird 60 min. unter N₂-Überschichtung dispergiert (Rührer 20 U/min., Ultraturrax 8000 U/min.).

Danach werden 2 mg Diazepam zu der Dispersion gegeben und 3,5 Stunden bei konstanter Temperatur von 50°C und einer Geschwindigkeit von 20 U/min. gerührt. Im Anschluß daran wird mit Hydratisierungsmedium auf 400 ml Endvolumen aufgefüllt.

### b) Herstellung überwiegend unilamellarer Diazepam-Liposomen

Die nach a) hergestellte Dispersion wird gemäß Bsp. 3b) mit Hilfe eines Extruders auf einen mittleren Teilchendurchmesser (Polydispersität 0,08) von 92 nm gebracht, in dem sie jeweils fünfmal durch Polycarbonat-Membranen (Fa. Nuclepore) mit den Porenweiten 200, 100 und 80 nm extrudiert wird. Die Diazepam-Konzentration der Liposomen-Dispersion beträgt 4,95 mg/ml.

### 9. Beispiel: Herstellung von Testosteron-haltigen Liposomen

### a) Dispergierung und Herstellung multilamellarer Testosteron-Liposomen

15 g Phosphatidylcholin aus Ei werden gemäß Bsp. 1 mit Hilfe eines Laborreaktors bei 25°C, 15 min. in 100 ml Hydratisierungsmedium (5 % Glucose-Lösung) dispergiert.

2 g Testosteron werden in 10 Portionen a 200 mg im Abstand von jeweils 15 min. in die Dispersion eingebracht. Die Dispersion wird dabei auf 50°C temperiert, die Rührergeschwindigkeit beträgt 20 U/min., die Ultraturraxgeschwindigkeit beträgt 1000 U/min.. Nach Abschluß der Wirkstoffzugabe wird noch 3 Stunden bei 50°C und einer Geschwindigkeit von 20 U/min. gerührt und mit Hydratisierungsmedium auf ein Volumen von 150 ml aufgefüllt.

### b) Herstellung überwiegend unilamellarer Testosteron-Liposomen

Die in a) hergestellte Dispersion multilamellarer Testosteron-Liposomen wird gemäß Bsp. 1b) 1 Stunde bei 750 bar homogenisiert. Produktausgang und Vorlagegefäß werden dabei auf 25°C temperiert. Die entstehenden unilamellaren Liposomen haben einen mittleren Teilchendurchmesser von 78 nm, eine Polydispersität von 0,21 und eine Testosteron-Konzentration von 9,87 mg/ml Dispersion.

## Patentansprüche

1. Verfahren zur Verkapselung fester oder flüssiger lipophiler Wirkstoffe zu diesen Wirkstoff enthaltenden Phospholipid-Liposomen aus einem Phospholipid oder Phospholipid-Gemisch mit mindestens einem lipophilen Wirkstoff in einem Hydratisierungsmedium, umfassend einen ersten Schritt, in dem man multilamellare, wirkstoffhaltige Liposomen erzeugt und einen zweiten Schritt, in dem man die mittlere Größe der wirkstoffenthaltenden Phospholipid-Liposomen durch übliche Verfahren auf eine Größe von < 200 nm einstellt, dadurch gekennzeichnet, daß man in dem ersten Schritt das Phospholipid oder Phospholipid-Gemisch unter Rühren in der Hälfte bis einem Dreiviertel der Menge des Hydratisierungsmediums unter Schutzgas dispergiert und nach Beendigung dieses Vorgangs auf eine Temperatur oberhalb der Phasenübergangstemperatur des Phospholipids oder Phospholipid-Gemisches temperiert, bei welcher man den Wirkstoff der Dispersion zugibt sowie den noch verbliebenen Teil des Hydratisierungsmediums auffüllt und man anschließend noch so lange bei dieser Temperatur weiterrührt, bis durch übliche Analysenmethoden kein freier Wirkstoff im Hydratisierungsmedium mehr nachgewiesen werden kann, wobei das Hydratisierungsmedium frei von organischen Lösungsmitteln und/oder Detergentien ist.

2. Verfahren nach Anspruch 1, dadurch gekennzeichnet, daß man das Phospholipid oder Phospholipid-Gemisch und/oder den Wirkstoff in Gegenwart eines Hilfsstoffes dispergiert.

3. Verfahren nach Anspruch 1 oder 2, dadurch gekennzeichnet, daß natürliche, halbsynthetische oder synthetische Phospholipide oder Gemische daraus eingesetzt werden.

4. Verfahren nach Ansprüchen 1 bis 3, dadurch gekennzeichnet, daß Phospholipide aus Soja und/oder Ei und/oder anderen natürlichen Materialien eingesetzt werden.

5. Verfahren nach Ansprüchen 1 bis 4, dadurch gekennzeichnet, daß die eingesetzten Phospholipide natürlichen Ursprungs durch katalytische Hydrierung chemisch modifiziert sind und verschiedene Hydrierungsgrade besitzen.

6. Verfahren nach Ansprüchen 1 bis 4, dadurch gekennzeichnet, daß als Phospholipide Phosphatidylcholin (PC), Phosphatidylethanolamin (PE), Phosphatidylglycerol (PG), Phosphatidylinosit (PJ), Phosphatidylserin (PS), Phosphatidsäure (PA), Di-palmitoyl-phosphatidylcholin (DPPC), Di-stearoyl-phosphatidylcholin (DSPC), Di-palmitoyl-phosphatidylethanolamin (DPPE), Di-stearoyl-phosphatidylethanolamin (DSPE), Di-myristoyl-phosphatidylserin (DMPS), Di-oleyl-phosphatidylcholin (DOPC) oder Gemische daraus eingesetzt werden.

7. Verfahren nach Ansprüchen 1 bis 6, dadurch gekennzeichnet, daß als Hilfsstoff C₄-C₂₂-Fettsäure-C₁-C₄-Alkylester und/oder Antioxidantien und/oder Verbindungen zur Erzeugung einer Oberflächenladung auf den Liposomen und/oder Verbindungen zur Stabilisierung des Bilayers eingesetzt werden.

8. Verfahren nach Ansprüchen 1 bis 7, dadurch gekennzeichnet, daß als Hydratisierungsmedium eine physiologisch verträgliche, isotonische Lösung eingesetzt wird.

9. Verfahren nach Ansprüchen 1 bis 8, dadurch gekennzeichnet, daß als Wirkstoffe Antibiotika, Hormone, Steroide, Fungizide, Tranquilizer, antineoplastische Substanzen, Immunsuppressiva, Anaesthetika, Analgetika oder Gemische daraus eingesetzt werden.

10. Verfahren nach Ansprüchen 1 bis 9, dadurch gekennzeichnet, daß auf eine Temperatur zwischen 10 und 70°C temperiert wird.

11. Verfahren nach Ansprüchen 1 bis 10 zur Herstellung von Gentamycin-, Propanidid-, Fentanyl-, Diazepam-, Cyclosporin A- oder Amphotericin B-haltigen Liposomen.

## Claims

1. A process for encapsulating solid or liquid lipophilic active substances to form phospholipid liposomes containing said active substance from a phospholipid or phospholipid mixture with at least one lipophilic active substance in a hydrating medium, comprising a first step wherein multi-lamellar liposomes containing the active substance are produced, and a second step wherein the average size of the phospholipid liposomes containing the active substance is adjusted to a size of <200 nm using conventional procedures, characterized in that in the first step the phospholipid or phospholipid mixture is dispersed in from one half to three quarters of the amount of the hydrating medium under a protective gas and, upon completion of this operation, the resulting dispersion is allowed to assume a treatment temperature above the phase transition temperature of the phospholipid or phospholipid mixture, at which temperature the active substance is added to the dispersion, followed by replenishing with the remaining portion of the hydrating medium, whereupon stirring is continued at this temperature until the free active substance cannot be detected any more in the hydrating medium by common analytical methods, the hydrating medium being free from organic solvents and/or detergents.

2. The process according to claim 1, characterized in that the phospholipid or phospholipid mixture an/or the active substance is(are) dispersed in the presence of an auxiliary material.

3. The process according to claims 1 or 2, characterized in that natural, semi-synthetic or synthetic phospholipids or mixtures thereof are used.

4. The process according to claims 1 to 3, characterized in that phospholipids from soybean and/or egg and/or other natural sources are employed.

5. The process according to claims 1 to 4, characterized in that the phospholipids employed of natural origin have been chemically modified by catalytic hydrogenation and possess various degrees of hydrogenation.

6. The process according to claims 1 to 4, characterized in that phosphatidyl choline (PC), phosphatidyl ethanolamine (PE), phosphatidyl glycerol (PG), phosphatidyl inositol (PJ), phosphatidyl serine (PS), phosphatide acid (PA), di-palmitoyl phosphatidyl choline (DPPC), di-stearoyl phosphatidyl choline (DSPC), di-palmitoyl phosphatidyl ethanolamine (DPPE), di-stearoyl phosphatidyl ethanolamine (DSPE), di-myristoyl phosphatidyl serine (DMPS), di-oleyl phosphatidyl choline (DOPC) or mixtures thereof are employed as the phospholipids.

7. The process according to claims 1 to 6, characterized in that C₄-C₂₂-fatty acid C₁-C₄-alkyl esters and/or antioxidants and/or compounds for generating a surface charge on the liposomes and/or compounds for stabilizing the bilayer are employed as the auxiliary material(s).

8. The process according to claims 1 to 7, characterized in that a physiologically compatible isotonic solution is used as the hydrating medium.

9. The process according to claims 1 to 8, characterized in that antibiotics, hormones, steroids, fungicides, tranquilizers, antineoplastic substances, immunosuppressants, anaesthetics, analgesics or mixtures thereof are employed as the active substances.

10. The process according to claims 1 to 9, characterized in that the treatment temperature is between 10 °C and 70 °C.

11. The process according to claims 1 to 10 for preparing liposomes containing gentamycin, propanidid, fentanyl, diazepam, cyclosporin A or amphotericin B.

## Revendications

1. Procédé pour encapsuler des principes actifs lipophiles, solides ou liquides, pour obtenir des liposomes de phospholipides contenant ce principe actif, à partir d'un phospholipide ou d'un mélange de phospholipides et d'au moins un principe actif lipophile, dans un milieu d'hydratation, qui comprend une première étape dans laquelle on produit des liposomes multilamellaires contenant un principe actif, et une deuxième étape, dans laquelle on ajuste à une valeur inférieure à 200 nm, par des procédés usuels, la granulométrie moyenne des liposomes de phospholipide contenant un principe actif, caractérisé en ce que, dans la première étape, on disperse le phospholipide ou le mélange de phospholipides, sous agitation et sous atmosphère d'un gaz protecteur, dans une quantité comprise entre la moitié et les trois quarts de la quantité du milieu d'hydratation, et que, après la fin de cette opération, on porte l'ensemble à l'équilibre de température à une température supérieure à la température de transition de phase du phospholipide ou du mélange de phospholipides, température à laquelle on ajoute le principe actif à la dispersion et on complète avec la partie encore restante du milieu d'hydratation, puis on agite encore, à cette température, jusqu'à ce que les méthodes d'analyse usuelles ne permettent plus de déceler de principe actif libre dans le milieu d'hydratation, le milieu d'hydratation étant exempt de solvants organiques et/ou de détergents.

2. Procédé selon la revendication 1, caractérisé en ce qu'on disperse le phospholipide ou le mélange de phospholipides et/ou le principe actif en présence d'un adjuvant.

3. Procédé selon la revendication 1 ou 2, caractérisé en ce qu'on utilise des phospholipides naturels, semi-synthétiques ou synthétiques, ou leurs mélanges.

4. Procédé selon les revendications 1 à 3, caractérisé en ce qu'on utilise les phospholipides du soja et/ou de l'oeuf et/ou d'autres substances naturelles.

5. Procédé selon les revendications 1 à 4, caractérisé en ce que les phospholipides utilisés, d'origine naturelle, subissent une modification chimique par hydrogénation catalytique et présentent différents degrés d'hydrogénation.

6. Procédé selon les revendications 1 à 4, caractérisé en ce qu'on utilise comme phospholipides la phosphatidylcholine (PC), la phosphatidyléthanolamine (PE), le phosphatidylglycérol (PG), le phosphatidylinositol (PJ), la phosphatidylsérine (PS), l'acide phosphatidique (PA), la dipalmitoyl-phosphatidylcholine (DPPC), la distéaroyl-phosphatidylcholine (DSPC), la dipalmitoyl-phosphatidyléthanolamine (DPPE), la distéaroyl-phosphatidyléthanolamine (DSPE), la dimyristoyl-phosphatidylsérine (DMPS), la di-oléyl-phosphatidylcholine (DOPC) ou leurs mélanges.

7. Procédé selon les revendications 1 à 6, caractérisé en ce qu'on utilise comme adjuvant un ester alkylique en C₁-C₄ d'un acide gras en C₄-C₂₂ et/ou des antioxydants et/ou des composés destinés à produire une charge superficielle sur les liposomes, et/ou des composés pour stabiliser la bicouche.

8. Procédé selon les revendications 1 à 7, caractérisé en ce qu'on utilise comme milieu d'hydratation une solution isotonique physiologiquement tolérée.

9. Procédé selon les revendications 1 à 8, caractérisé en ce qu'on utilise comme principes actifs des antibiotiques, des hormones, des agents stéroïdiens, des fongicides, des tranquillisants, des substances anti-néoplasiques, des immunosuppresseurs, des anesthésiques, des analgésiques ou leurs mélanges.

10. Procédé selon les revendications 1 à 9, caractérisé en ce qu'on porte à l'équilibre de température à une température de 10 à 70°C.

11. Procédé selon les revendications 1 à 10, pour préparer des liposomes contenant de la gentamycine, du propanidide, du fentanyl, du diazépam, de la cyclosporine A ou de l'amphotéricine B.
